# EUROPEAN PATENT APPLICATION

(11) **EP 3 916 384 A1**
(43) Date of publication of application: **01.12.2021**
(21) Application number: 21175239.9
(22) Date of filing: 21.05.2021
(51) Int. Cl.: G01N 33/38, G01N 27/22, G01N 27/48

(54) **MEASUREMENT DEVICE TO MEASURE TEMPERATURE AND HUMIDITY IN A STRUCTURAL ELEMENT**

(30) Priority: 25.05.2020 FI 20205527
(71) Applicant: KUI Technologies Oy, 00100 Helsinki (FI)
(72) Inventor: PARRU, Timo, 00100 Helsinki (FI); SUOMI, Heli, 00100 Helsinki (FI)
(74) Representative: Berggren Oy

(57) **Abstract**

An inventive structural measurement device is for measuring temperature and/or humidity inside a structural element. The measurement device comprises a rod (2), which is arranged to be inserted into the structural element, and a base (3), which is arranged to be against a surface of the structural element. The rod (2) contains only the sensor modules (4), connection line (5A, 5B, 5C) in order to connect the sensor modules to the base, holes (6) in order to connect the sensor modules (4) to the structural element in an open way, and possibly at least one block (20) in order to separate two of said sensor modules into separate sections inside the rod.

## Description

### Field of technology

The invention relates to a structural measurement device. The structural measurement device is used to measure temperature and/or humidity inside a structural element.

### Prior art

A structural measurement device is used to measure temperature and/or humidity inside a structural element. A known measurement device on market has a rod, which is arranged to be inserted into the structural element. The rod has sensor modules to measure temperature and humidity inside the structural element. Further, the rod has batteries inside the rod. The tip and a part of the rod has a smaller diameter than the main part of the rod containing the batteries. The device has also separate sleeve and a gasket ring. The sensor modules 4 are near both ends of the rod and in a middle part of the rod. The measurement device is also arranged transmit measurement results to a cloud service. The measurement device can be used in walls, base floors and roofs. US 20090211357 shows a device for sensing a condition, such as humidity, through an opening in a surface. The device has a circuit board with electronic and where sensors have been connected. The circuit board has two parts connected with each other through a connector. US 2014/0216143 discloses a device, which is used to measure moisture condition during casting of a building structure. Although, the measurement devices known on market works properly, a more accurate measurement device would be beneficial. Other known measurement devices are used on the surface of a structural element without inserting into the structural element.

### Short description

The object of the invention is to provide a structural measurement device, which provides more accurate measurements than known solutions. The idea of the invention is that the structure of the inventive measurement device disturbs as little as possible the functioning of measured structure. The object of the invention is achieved in a way described in the independent claims. Dependent claims illustrate different embodiments of the invention.

An inventive structural measurement device is for measuring temperature and/or humidity inside a structural element. The measurement device comprises a rod, which is arranged to be inserted into the structural element, and a base, which is arranged to be against a surface of the structural element. The rod has sensor modules near both ends of the rod and in a middle part of the rod. The measurement device further comprises communication means to transmit measurements of the sensor modules. The rod has a tip part having a diameter that corresponds with the diameter of the rod. The rod contains only the sensor modules, connection lines in order to connect the sensor modules to the base, holes in order to connect the sensor modules to the structural element in an open way, and possibly at least one block in order to separate two of said sensor modules into separate sections inside the rod. The base of the structural measurement device comprises a circuit board being connected to the sensor modules through the connection lines. In addition, the base comprises the antenna connected to the circuit board and a replaceable battery connected to the circuit board.

### List of figures

In the following, the invention is described in more detail by reference to the enclosed drawings, where
- Figure 1: illustrates an example of a structural measurement device connection,
- Figure 2: illustrates an installation example of the structural measurement device,
- Figure 3: illustrates another installation example of the structural measurement device,
- Figure 4: illustrates another example of a structural measurement device,
- Figure 5: illustrates yet another example of a structural measurement device,
- Figure 6: illustrates an example of a base of the structural measurement device, and
- Figure 7: illustrates another example of a base of the structural measurement device.

### Description of the invention

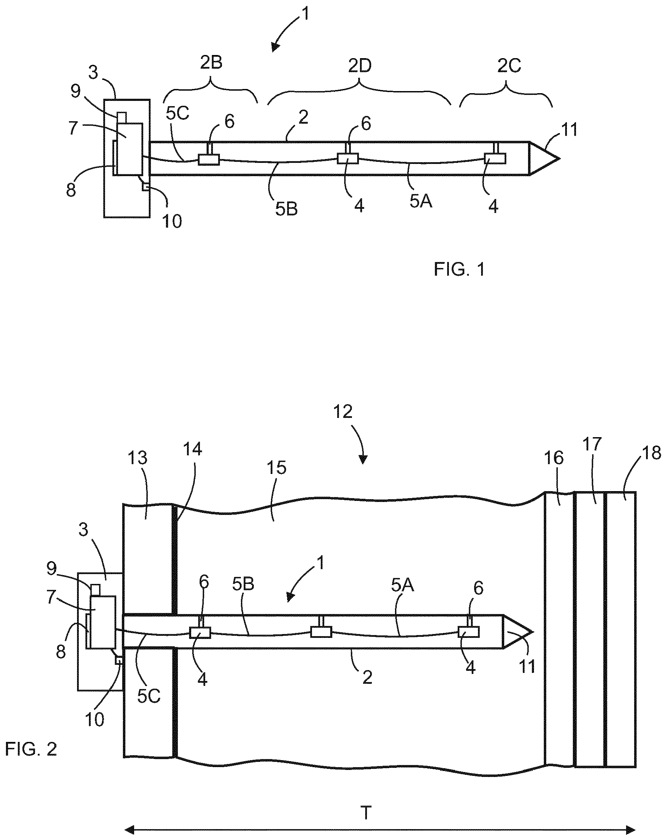

Figure 1 illustrates an example of the inventive structural measurement device 1 for measuring temperature and/or humidity inside a structural element. The measurement device comprises a rod 2, which is arranged to be inserted into the structural element, and a base 3, which is arranged to be against a surface of the structural element. Figure 2 shows an installation example where the measurement device 1 has been installed into the structural element 12, which is a wall in this case.

The rod 2 has sensor modules 4 near both ends 2B, 2C of the rod and in a middle part 2D of the rod. The embodiment of figure 1 has three sensor modules 4 which are capable to measure temperature and humidity, but they may also be more sensor modules in the rod if desired. So, the sensor module can be capable of measuring temperature and humidity both, but they can be capable of measuring only temperature or humidity. In figure 1, the sensor module 4 near the tip 11 can be called as an outer sensor module, and the sensor near the base 3 can be called as an inner sensor module. The sensor module between these sensor modules can be called as a middle sensor module. In another embodiment where temperature and humidity are measured by separate sensor modules 4, there can be two sensor modules near the tip 11, so there can be two outer sensor modules. The same applies with the middle sensor modules and the inner sensor modules.

The measurement device further comprises an antenna 9 to transmit measurements of the sensor modules. The rod has a tip part 11 having a diameter that corresponds with the diameter of the rod. In this way the installation into the wall 12 or into another structural element is easy.

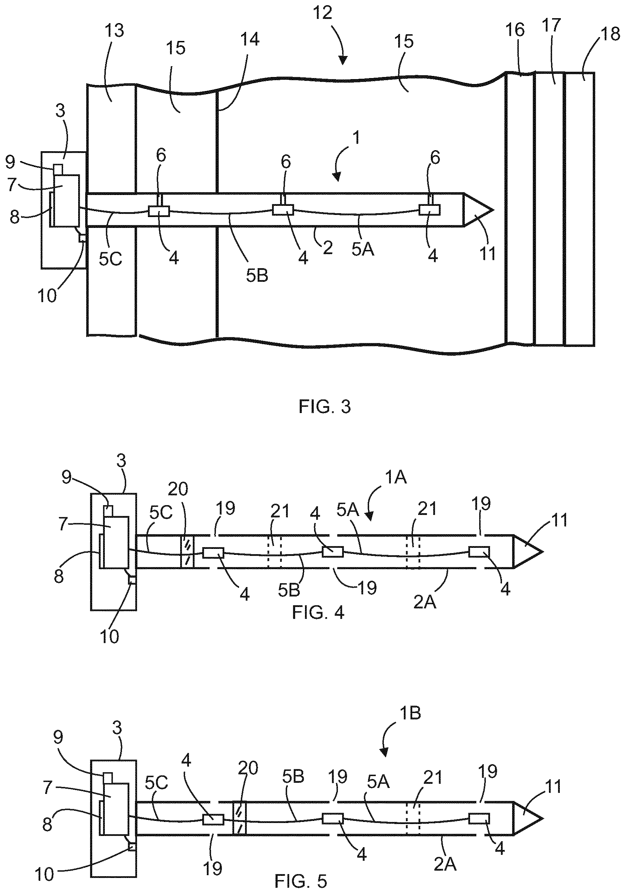

The rod 2 contains only the sensor modules 4, connection lines 5A, 5B, 5C in order to connect the sensor modules to the base 3, holes 6 in order to connect the sensor modules to the structural element in an open way, and possibly at least one block 20, 21 (See figure 4.) in order to separate two of said sensor modules 4 into separate sections inside the rod 2A. In this way measurements of the sensor modules 4 are disturbed as less as possible. Alternatively, tube is filled with thermal insulation material between the sensor modules in order to prevent air moving inside the tube and so that inside tube has same thermal insulation as outside tube. The base 3 of the structural measurement device comprises a circuit board 7 being connected to the sensor modules 4 through the connection lines 5A, 5B, 5C. In addition, the base comprises the antenna 9 connected to the circuit board 7 and a replaceable battery 8 connected to the circuit board. The antenna can be also be a printed on the circuit board, so the connection to the circuit board is provided by the integral structure. Since the circuit board 7, the battery 8, and the antenna 9 are situated in the base 3 they do not disturb/interfere much the measurements made by the sensor modules 4 in the rod 2, especially when compared with known solutions having at least some of these situated into the rod. ) So, the measurements and also the functioning of the structural element are not disturbed by conducting heat because metal and plastic parts are minimized in the rod, and by heating up the structural element, like the wall because the most power consuming parts (circuit board components like a processor, and transmitter/receiver components, and the antenna) are outside the rod.

As said figure 2 shows the example of the wall wherein the measurement device 1 has installed. On the inner side the wall 12 has gypsum board 13 and below it a vapour barrier 14. In the middle of the wall there is thermal insulation 15, which is with a timber frame inside the wall. So, the example of figure 2 shows a timber framed wall structure. On the outer side of the wall there are external cladding 18 (like timber cladding), a ventilator slot 17, and a wind shield board 16.

Outdoor atmosphere changes with seasons and varies over a 24-hour period. Indoor atmosphere does not usually change as much as outdoor atmosphere. So, the outdoor temperature and humidity especially affect to temperature and humidity inside the wall structure or inside another structural element. For example, in winter, the outdoor temperature can be -20 Celsius, and relative humidity, RH, 90%. At the same time, the indoor temperature is +20 Celsius and the relative humidity about. 30 - 40 %. It is worth to note that the RH value is a relative value indicating relatively the amount of water vapour in the air relating to the maximum amount of water vapour that the air can hold in the same temperature. In winter, the inside air contains more water than the outside air. Therefore, a vapour barrier 14 may be used in a structural element, like a wall.

Considering the winter condition said above, the temperature changes inside the wall structure from -20 Celsius outdoor to +20 Celsius indoor. The most part of the temperature change happens inside the thermal insulation 15. The humidity condition inside the wall structure changes also. Since the indoor air contains more water vapour than the cold outdoor air, the partial pressure of the indoor water vapour keeps pushing water vapour into the wall. The vapour barrier hinders this movement of the water vapour.

In order to check that the wall structure or another structural element works fine, the inventive structural measurement device measures temperature and/or humidity inside a structural element at least in three different locations in the thickness direction T of the wall or the other structural element. The allowed and not-allowed temperature and humidity conditions in different locations inside the structural element can be calculated beforehand. Since the locations of the sensor modules in the measurement device are fixed, their locations inside the wall or the other structural element is known also when installed into the element. When comparing the measured temperature and humidity values with the calculated values, it can be detected how the structural element works. The comparison and the said calculation can be made in a cloud service, more precisely in a server of a measurement service provider. The connection to the cloud service or another external arrangement or device is made through the antenna 9. If the comparison reveals that the temperature and/or humidity values are not-allowed inside the structural element, the owner/maintenance of the structural element, i.e. the owner/maintenance of the building comprising the structural element, can be alerted in order to perform necessary actions. For example, the reason for the not-allowed humidity values can be a broken vapour barrier, which should be repaired, or a leak on a water pipeline.

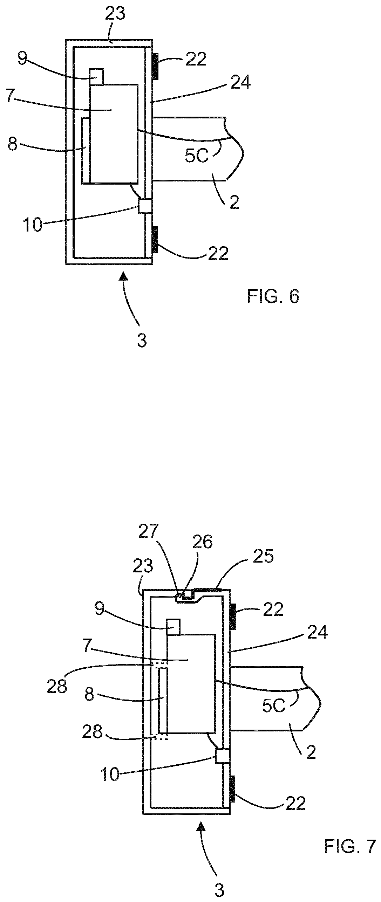

As said, the base 3 contains the circuit board 7, the replaceable battery 8, and the antenna 9. The structural measurement device can transmit the measurement to the said cloud service or to another receiver by utilizing the antenna 9. The other receiver can also be, for example, a reader utilizing an RF technology or a NFC technology. When these devices are situated in the base 3, they do not disturb so much the sensor modules 4 in the rod 2. Figure 6 a more detailed example of the base 3. The base may have an adhesive ring 22, which can be used to attach the base 3 on the surface of the structural element. The body of the base can be made two separate part, a base plate 24, and a cup 23, The cup can be attached to the base plate in such a way that in can be removed in order to make it possible to change the battery. Alternatively, the cap 23 may have a separate lid. In addition, as illustrated in the figures the base 3 may also contain a separate sensor module 10 to measure temperature and/or humidity at the vicinity of the base. Since the installation is normally made inside a building to the structural element, the separate sensor module 10 usually measures the inside temperature and/or humidity in the building. However, it is also possible to insert the structural measurement device 1 to the structural element from the outer side. Thus, the base 3 (the cap/base hole) may have at least one hole for the separate sensor module. It is also possible that as shown in the figures 6 and 7 that the sensors module is against the wall via the hole of the base when installed. The cup 23 protects the separate sensor module 10 from different disturbances in the air (inside air), like an open window or a heating unit nearby, or air flow on the surface of the wall. Therefore, the separate sensor may be arranged to measure the temperature of the inner wall as accurate as possible. It is also possible that the base 3 has two sensor modules, one to measure the temperature of the wall and the other sensor module to measure temperature/humidity around the base 3 (like the inside temperature and/or humidity).

Figure 7 shows another example of the base 3. The base plate 24 has one or two places 25 where a user can press the lid in order to move a clip/s 26 for removing the cup 23. The cup has a slot/s 27 for the clip/s in order to keep the cup with the base plate 24. When the cup is removed, the battery 8 can be replaced. After changing the battery, the cup is attached back on the base plate. The cup 23 may have also fastening projections 28 for the battery. So, the battery is attached removeable with the cup in such a way that battery is also in electrical connection with the circuit board 7. Another way is that the battery is removeable attached on the circuit board. The rod is not taken away from the structural element when changing the battery.

Figure 3 shows another installation example. Here the vapour barrier 14 is situated deeper in the wall 12 in order that it is not broken so easily if pictures, painting or other items are hung on the wall. In this case the inner sensor module 4 may be on the inner side with respect to the vapour barrier 14.

Figures 1 - 3 show the embodiment wherein the rod 2 a has solid structure, but the structure can also be hollow. Figures 4 and 5 illustrates embodiments 1A, 1B, having the hollow rod 2A. The cross-section of the rod is circle in different embodiments of the invention. The circle cross-section of the rod is beneficial when inserting the rod into the structural element. The circle cross-section makes it also possible that the vapour barrier can be tightly against the surface of the rod. Therefore, the installation of the structural measurement device does not deteriorate the functioning of the vapour barrier 14.

The embodiments of figures 4 and 5 with the hollow structure rod 2A has at least one block 20, 21 in order to separate two of said sensor modules 4 into separate sections inside the rod. One block 20 is arranged to be in line with a vapour barrier 14 of the structural element, like the wall 12, when the measurement device is installed into the structural element. This block 20 works as a vapour barrier inside the rod 2A. The embodiment of figure 4 has been designed to be used with a wall structure wherein the vapour barrier 14 is right behind the inner board (usually a gypsum board) like figure 2 illustrates. The embodiment of figure 5 has been designed to be used with a wall structure wherein the vapour barrier 14 deeper in the insulation material of the wall like figure 3 illustrates.

When there are further blocks 21, they separate the sensor modules 4 in the longitudinal direction of the rod 2A as illustrated in figures 4 and 5. In this way each sensor module location in the rod has its own section and measurements of sensor modules can be more accurate. The blocks prevent the air movement and heat movement along the rod. As said, the rod could also be filled with insulation material.

The holes 6, 19 for connecting the sensor modules to the structural element in an open way perform direct and free connection for the sensor modules 4 to measure temperature and/or humidity of the structural element near the sensor module. As can be seen in the figures the measurements are made in at least three separate locations in the thickness direction T of the other structural element. The sensor module comprises a sensor piece and connections to the connection lines. In addition, the sensor module may comprise simple components like a resistor and a capacitor.

The rod is polyurethane, ABS plastic, PC plastic or other suitable plastic. Polyurethane is suitable for the solid rod, but it can also be used with the hollow structure rod. The hollow structure rod may also have a net structure when the meshes of the net structure provide said holes 19 for the sensor modules 4. The net structure can also made using said plastic materials. The material of the rod has low thermal conductivity.

The rod may also comprise a separate strip which can be inserted into the rod and forms a part of the rod. The separate strip can be used for attaching the connection lines and the sensor modules onto the strip. The strip supports also the other rod structure. Manufacturing of the measurement device can be easier when using the strip or corresponding rod part. The strip or the corresponding part can be polyurethane, ABS plastic, PC plastic or other suitable plastic. When attached with the rest of the rod the strip does not disturb the measurements of the sensor modules any more than the rest of the rod structure.

The connection lines 5A, 5B, 5C can be direct wires between the circuit board 7 and each sensor module 4. The connection lines can also be implemented as a communication bus. For example, an 12C communication bus can be used having lines for a ground, data, time and operating voltage.

The tip part 11 can be a separate part connected to the rod 2, 2A, or an integral part of the rod. The separate part may be convenient regarding manufacturing. So, the tip part 11 can be the same material as the rod or different suitable material like polyurethane, ABS plastic, PC plastic or other suitable plastic. The base 3 can also be made with the same material as the rod or other suitable material, like polyurethane, ABS plastic, PC plastic or other suitable plastic. The pike of the tip part should be relatively sharp in order to make installation of the measurement device into a structural element easier.

When installing the measurement device, for example into the wall having a vapour barrier, a small diameter bore (for example 3 mm) is made through the inner plate 13 and the vapor barrier 14. The diameter of the rod 2, like 5 mm - 10 mm, (and the tip part) is larger than the diameter of the initial bore. Then the bore in the inner plate is enlarged to match with the diameter of the rod 2, 2A, without increasing the bore hole on the vapor barrier 14. The vapour barrier is normally a suitable plastic sheet. When inserting the measurement device, the tip part 11 goes through the hole in the vapour barrier and at the same time increases the hole in such a way that the vapour barrier 14 is tightly against the tip part 11 and finally tightly against the rod 2, 2A. The installation is easy, and the performance of the vapour barrier remains as it was designed.

As said the inventive structural measurement device provides more accurate measurement results than known solutions. The rod of the measurement device contains the sensor modules, their connection lines, and possible block/s. The circuit board, antenna and the battery are situated outside the rod, in the base 3. The material of the rod is polyurethane, ABS plastic, PC plastic or other suitable plastic which do not interfere with the measurements of the sensor modules. So, the structure of the inventive device interferes with the measurements of the sensor modules as less as possible.

The inventive structural measurement device can used in most types of building like homes, apartment blocks, commercial buildings etc. The device can be installed to walls, roofs, and floors. Using several measurement device conditions of all structural elements of the building can be monitored.

It is evident from the above that the invention is not limited to the embodiments described in this text but can be implemented in many other different embodiments within the scope of the independent claims.

## Claims

1. A structural measurement device (1) to measure temperature and/or humidity inside a structural element, the measurement device comprising a rod (2) being arranged to be inserted into the structural element, and a base (3) being arranged to be against a surface of the structural element, the rod having sensor modules (4) near both ends of the rod and in a middle part of the rod, the measurement device further comprising an antenna (9) to transmit measurements of the sensor modules, **characterised in that** the rod (2) has a tip part (11) having a diameter that is corresponding with the diameter of the rod (2), the rod containing only the sensor modules (4), connection lines (5A, 5B, 5C) in order to connect the sensor modules to the base (3), holes (6, 19) in order to connect the sensor modules to the structural element in an open way, and blocks (20, 21) in order to separate said sensor modules (4) and said holes (6, 19) into separate sections inside the rod,
the base (3) comprising a circuit board (7) being connected to the sensor modules through the connection lines (5A, 5B, 5C), the base further comprising the antenna (9) connected to the circuit board (7) and a replaceable battery (8) connected to the circuit board.

2. A structural measurement device according to claim 1, **characterised in that** rod (2, 2A) has a hollow structure or a solid structure.

3. A structural measurement device according to claim 2, **characterised in that** the rod (2, 2A) is polyurethane, ABS plastic, PC plastic or other suitable plastic.

4. A structural measurement device according to claim 3, **characterised in that** the hollow structure rod (2A) has also a net structure.

5. A structural measurement device according to claim 2 or 3, **characterised in that** the hollow structure rod (2A) has at least one block (20, 21) in order to separate two of said sensor modules (4) into separate sections inside the rod.

6. A structural measurement device according to claim 5, **characterised in that** one block (20) is arranged to be in line with a vapour barrier of the structural element when the measurement device is installed into the structural element.

7. A structural measurement device according to claim 1 or 2, **characterised in that** the tip part (11) is a separate part connected to the rod (2, 2A), or an integral part of the rod.

8. A structural measurement device according to any of claim 2 -7, **characterised in that** the connection lines are implemented as a communication bus.

9. A structural measurement device according to any of claim 2 - 8, **characterised in that** the rod (2, 2A) comprises a separate strip.

10. A structural measurement device according to any of claim 2 - 9, **characterised in that** the base (3) comprises also a separate sensor module (10).
